Europäisches Patentamt

European Patent Office

Office européen des brevets

(11) Publication number: **0 077 261**
**B1**

## (12) EUROPEAN PATENT SPECIFICATION

(45) Date of publication of patent specification: **29.07.87**

(21) Application number: **82401833.7**

(22) Date of filing: **06.10.82**

(51) Int. Cl.⁴: **A 61 K 9/00,** A 61 K 47/00,
C 08 L 29/04, C 08 L 1/26

(54) Biosoluble ocular insert.

(30) Priority: **08.10.81 US 309852**

(43) Date of publication of application:
**20.04.83 Bulletin 83/16**

(45) Publication of the grant of the patent:
**29.07.87 Bulletin 87/31**

(84) Designated Contracting States:
**AT BE CH DE FR GB IT LI LU NL SE**

(56) References cited:
**EP-A-0 010 876**
**GB-A-1 359 198**
**GB-A-1 360 927**
**US-A-3 968 201**

(73) Proprietor: **MERCK & CO. INC.**
**126, East Lincoln Avenue P.O. Box 2000**
**Rahway New Jersey 07065 (US)**

(72) Inventor: **V. Bondi, Joseph**
**3825 Kratz Road**
**Collegeville Pennsylvania 19426 (US)**
Inventor: **Harwood, Richard J.**
**3219 Adams Drive**
**Bensalem Pennsylvania, 19020 (US)**

(74) Representative: **Martin, Jean-Jacques et al**
**Cabinet REGIMBEAU 26, Avenue Kléber**
**F-75116 Paris (FR)**

Courier Press, Leamington Spa, England.

**Description**

This invention is concerned with ocular inserts, with or without a pharmacologically active agent, comprising at least 15% by weight of polyvinyl alcohol with a molecular weight of 2000 to 3000, and from 1 to 10% by weight of glycerin, which do not cause blurred vision and which when formulated with enteric coating materials as a constituent of the matrix provide a means for controlling and predicting dissolution rates for slow release and prolonged therapeutic effect.

Solid ocular inserts are now well known in the art and they are generally a great advance over the prior liquid and ointment forms of medication. However, the prior art solid biosoluble ocular inserts frequently caused blurred vision because the high molecular weight polymers commonly employed altered the viscosity and refractive index of the tear film. For example, GB—A—1 359 198 teaches the use of cross-linked polyvinyl alcohol. These changes resulted in the formation of "Schlieren" or discontinuities in the precorneal tear film. The prior art solid biosoluble inserts also served to augment the blurring by the presence of a large volume of the gelatinized, hydrated polymers which tended to fracture into fragments due to blinking, giving rise to a potentially more rapid dissolution.

Now with the present invention there is provided ocular inserts that minimize the blurred vision effect by providing materials that do not materially change the viscosity and index of refraction of the tear film and optionally provide those materials at a rate sufficiently slowly as to provide only low volumes of the solubilized polymers. In addition, the compositions described do not form gelatinous masses in the cul-de-sac.

Description of the invention

The novel non-vision blurring ocular insert of this invention comprises a number of different components in various relative amounts and may or may not include a pharmacologically active agent. These components are:

Polyvinyl alcohol

This component is one of the principal matrix materials giving the insert its desired shape and integrity. The particular polyvinyl alcohol useful in the novel insert has a molecular weight between 2000 and 3000 and forms at least 15% by weight of the finished product.

Plasticizer

The ocular inserts prepared in accordance with this invention also contain glycerin to make the ophthalmic inserts more pliable. Glycerin is present in amounts of from 1.0% to 10% by weight.

Hydroxypropyl cellulose

The hydroxypropyl cellulose useful in the formulation of this invention is of low molecular weight from 30,000 to 100,000. It is an optional component and is present in amounts from 0—50% by weight of the finished insert.

Polyvinylpyrrolidone

Polyvinylpyrrolidone is an optional component present in amounts from 0 to 20% by weight and its presence or absence is dictated by the nature of the pharmacologically active agent that may be included. It complexes with certain materials such as indomethacin and is known to bind with certain tissue and thus has the potential of creating a depot type of medication.

Monosaccharide alcohol

Mannitol, sorbitol, or any of the other sugar alcohols, but especially mannitol, optionally may be included as a component of the insert as a diluent up to 30% by weight of the total.

Enteric coating polymer

An optional component is one of the several known enteric coating polymers such as hydroxypropylmethylcellulose phthalate, cellulose acetate phthalate, hydroxypropylmethyl cellulose acid succinate, or acrylic acid polymer, which is not present as a coating, but rather as a component of the matrix, being homogeneously distributed therein. It constitutes up to 100% by weight of the total and serves to provide a slow even release of pharmacologically active agent or a slow-release source of the polymer itself or the other components such as polyvinyl alcohol and hydroxypropylmethylcellulose. Incorporation of one of the enteric coating materials extends the dissolution rate from about 15 minutes up to about 24 hours, depending on the relative amount of the material.

Pharmacologically active agent

If the novel ocular insert of this invention is intended for delivery of a pharmacologically active agent it is incorporated therein in an amount up to 99% by weight thereof. Suitable drugs for use in therapy with the ocular drug delivery system of the invention include, without limitation, drugs that produce a physiologically or pharmacologically localized or systemic effect in animals, including warm blooded

2

mammals, human and primates, valuable domestic household, sport or farm animals such as horses, dogs, cats, cattle, sheep and the like; or for administering to laboratory animals such as mice, monkeys, rats, rabbits and guinea pigs. The active drugs that can be administered by the novel drug delivery system of the invention include, without limitation: antibiotics such as tetracycline, chlortetracycline, bacitracin, neomycin, polymyxin, gramicidin, oxytetracycline, chloramphenicol, gentamycin, and erythromycin; antibacterials such as sulfonamides, sulfacetamide, sulfamethizole and sulfisoxazole; antivirals, including idoxuridine; and other antibacterial agents such as nitrofurazone and sodium propionate; anti-allergenics such as antazoline, methapyriline, chlorpheniramine, pyrilamine and prophenpyridamine; steroidal antiinflammatories such as hydrocortisone, hydrocortisone acetate, dexamethasone, dexamethasone 21-phosphate, fluocinolone, medrysone, prednisolone, methylprednisolone, prednisolone 21-phosphate, prednisonolone acetate, fluoromethalone, betamethasone and triamcinolone; non-steroidal antiinflammatories such as indomethacin, sulindac and aspirin; decongestants such as phenylephrine, naphazoline, and tetrahydrazoline; miotics and anticholinesterases such as pilocarpine, eserine, carbachol, di-isopropyl fluorophosphate, phospholine iodide, and demecarium bromide; mydriatics such as atropine sulfate, cyclopentolate homatropine, scopolamine, tropicamide, eucatropine, and hydroxyamphetamine; sympathomimetics such as epinephrine; and β-blockers such as propanolol and timolol.

Drugs contained in the insert can be in different forms, such as uncharged molecules, components of molecular complexes or non-irritating, pharmacologically acceptable derivatives thereof. Simple derivatives of the drugs such as pharmaceutically acceptable ethers, esters, amides, and the like which have desirable retention and release characteristics but which are easily hydrolyzed by body pH, or enzymes to active forms can be employed.

The amount of drug incorporated in the system varies depending on the drug, the desired therapeutic effect, and the time span for which the system provides therapy. Since a variety of systems in a variety of sizes and shapes are intended to provide dosage regimens for therapy for a variety of maladies, there is no critical upper limit on the amount of drug incorporated in the system. The lower limit too will depend on the activity of the drug and the time span of its release from the system. Thus, it is not practical to define a range for the therapeutically effective amount of drug to be incorporated in or released by the system. However, the amount of drug present is generally nonlimited and it is an amount equal to or larger than the amount of drug that on its release from the system is effective for bringing about the drug's effects. Generally, an ocular system containing a pharmacologically active agent will contain from 1 microgram to about 300 micrograms of drug or more, for releasing the drug to the eye at art recognized dosage rates.

The compositions of this invention can be prepared by various methods. Thus, the drug and the polymer(s) can be dissolved in a suitable solvent and the solution evaporated to afford a thin film comprising the polymer(s) and the drug which can then be subdivided to prepare suitable inserts containing the desired amount of the medicament. Alternatively, and in accordance with a preferred embodiment of our invention, we find that the inserts can be prepared most conveniently using the thermoplastic properties of the polymer(s) described above. For example, the medicament and the polymer(s) can be warmed together at temperatures between about 65.5°C and 204.4°C and the resulting mixture compression molded to form a thin film. It is generally preferred to prepare the inserts by compression molding or extrusion in accordance with procedures which are well known in the art. The compression molded or extruded product can then be subdivided to afford inserts of suitable size for administration in the eye. For example, castings or compression molded films having a thickness of about 0.5 mm. to 1.5 mm. can be subdivided to obtain suitable inserts in the form of squares, rectangles, circles, semi-circles, and the like containing the desired amount of active ingredient. Rectangular segments of the cast or compressed film having a thickness between about 0.5 and 1.5 mm. can be cut to afford shapes such as rectangular plates of 4×5—15 mm. or ovals of comparable size. Similarly, extruded rods having a diameter between about 0.5 and 1.5 mm. can be cut into suitable sections to provide the desired dosage of medicament. For example, rods of 1.0 to 1.5 mm. in diameter and up to 5 mm. long are found to be satisfactory. The inserts may also be directly formed by injection molding. All of the ophthalmic inserts prepared in accordance with the present invention should be formed so that they do not have any sharp edges or corners which could cause damage to the eye.

# 0 077 261

Example 1

The following table exemplifies 6 typical formulations with their respective dissolution times.

### Composition of six ophthalmic insert matrices
Mg. per 3 mg. insert

| Composition | A | B | C | D | E | G |
|---|---|---|---|---|---|---|
| Polyvinyl Alcohol | 1.50 | 0.45 | 1.50 | 2.10 | 1.50 | 1.5 |
| Hydroxypropyl cellulose EF | 0.60 | — | — | — | — | 0.60 |
| Hydroxypropyl methylcellulose phthalate | — | 2.25 | — | — | 1.35 | — |
| Polyvinylpyrrolidone | — | — | 0.60 | — | — | — |
| Mannitol | 0.75 | — | 0.75 | 0.75 | — | 0.55 |
| Glycerin | 0.15 | 0.30 | 0.15 | 0.15 | 0.15 | 0.15 |
| Timolol maleate | — | — | — | — | — | 0.20 |
| Estimated dissolution time *in vivo* based on *in vitro* and *in vivo* data in rabbits | 15 min. | 6—7 hrs. | 15 min. | 15 min. | 1—2 hrs. | 15 min |

Formulation A is prepared as follows:

Glycerin and mannitol are blended together to form a granular mass. Polyvinyl alcohol is gradually added until a uniform mixture is obtained. Finally, the hydroxypropyl cellulose is added and the entire mixture extruded using a twin screw extruder to form a strand 1 mm in diameter which can be cut into lengths of the desired size.

Example 2

Assay of vision blurring potential

An *in vivo* assessment of the blurred vision potential is made using dogs. An insert is placed into the cul-de-sac of a conditioned female beagle dog. Observations of the keratoscopic mires using a self illuminated placido disk (Klein Keratoscope) are then made at regular intervals until the insert is completely dissolved. The degree of distortion of the mires is graded on a scale of one to six. A plot of degree of distortion versus time is made and the area under the curve measured. This value is termed the distortion index. A second value obtained from the measurement is the duration of distortion which is defined as the total time that distortion occurs during a given experiment. For purposes of accuracy, each insert composition is tested in 3 animals.

4

**0 077 261**

Results

| Insert | Distortion index | Duration of distortion |
|---|---|---|
| 5 mg Hydroxypropyl cellulose HF | 415 | 120 min. |
| 3 mg Hydroxypropyl cellulose JF | 30 | 30 min. |
| 5 mg Polyvinyl alcohol 30—20 | 120 | 120 min. |
| 2.5 mg Hydroxypropyl cellulose EF | 297 | 180 min. |
| Matrix A of Example 1 | 5 | 10 min. |
| B of Example 1 | 0 | 0 min. |
| C of Example 1 | 0 | 0 min. |
| D of Example 1 | 8 | 10 min. |
| E of Example 1 | 2 | 5 min. |

**Claims**

1. A biosoluble non-vision-blurring ocular insert, characterized in that it comprises:
at least 15% by weight of polyvinyl alcohol with a molecular weight of 2000 to 3000, and from 1 to 10% by weight of glycerin.

2. A biosoluble non-vision-blurring ocular insert according to claim 1, characterized in that it also comprises up to 50% by weight of hydroxypropylcellulose with a molecular weight of 30,000 to 100,000.

3. A biosoluble non-vision-blurring ocular insert according to only one of claims 1 and 2, characterized in that it also comprises up to 20% by weight of polyvinylpyrrolidone.

4. A biosoluble non-vision-blurring ocular insert according to any one of claims 1 to 3, characterized in that it also comprises up to 30% mannitol.

5. A biosoluble non-vision-blurring ocular insert according to any one of claims 1 to 4, characterized in that it also comprises an enteric coating polymer, more particularly hydroxypropylmethylcellulose phthalate.

6. A biosoluble non-vision-blurring ocular insert according to any one of claims 1 to 5, characterized in that it also comprises an effective amount of a pharmacologically active agent.

7. A biosoluble non-vision-blurring ocular insert according to claim 1, characterized in that it weighs approximately 3 mg and is composed of 1.50 mg of polyvinyl alcohol, 0.6 mg of hydroxypropylcellulose EF, 0.75 mg of mannitol and 0.15 mg of glycerin.

8. A biosoluble non-vision-blurring ocular insert according to claim 1, characterized in that it weighs approximately 3 mg and is composed of 0.45 mg of polyvinyl alcohol, 2.25 mg of hydroxypropylmethylcellulose phthalate and 0.30 mg of glycerin.

9. A biosoluble non-vision-blurring ocular insert according to claim 1, characterized in that it weighs approximately 3 mg and is composed of 1.5 mg of polyvinyl alcohol, 0.60 mg of polyvinylpyrrolidone, 0.75 mg of mannitol and 0.15 mg of glycerin.

10. A biosoluble non-vision-blurring ocular insert according to claim 1, characterized in that it weighs approximately 3 mg and is composed of 2.10 mg of polyvinyl alcohol, 0.75 mg of mannitol and 0.15 mg of glycerin.

11. A biosoluble non-vision-blurring ocular insert according to claim 1, characterized in that it weighs approximately 3 mg and is composed of 1.50 mg of polyvinyl alcohol, 1.35 mg of hydroxypropylmethylcellulose phthalate and 0.15 mg of glycerin.

**Patentansprüche**

1. Biolöslicher, die Sicht nicht verschleiernder Augeneinsatz, dadurch gekennzeichnet, daß er umfaßt:
wenigstens 15 Gew.-% Polyvinylalkohol mit einem Molekulargewicht von 2000 bis 3000 und 1 bis 10 Gew.-% Glycerin.

2. Biolöslicher, die Sicht nicht verschleiernder Augeneinsatz nach Anspruch 1, dadurch gekennzeichnet, daß er auch bis zu 50 Gew.-% Hydroxypropylcellulose mit einem Molekulargewicht von 30 000 bis 100 000 umfaßt.

3. Biolöslicher, die Sicht nicht verschleiernder Augeneinsatz nach einem der Ansprüche 1 und 2, dadurch gekennzeichnet, daß er auch bis zu 20 Gew.-% Polyvinylpyrrolidon umfaßt.

5

4. Biolöslicher, die Sicht nicht verschleiernder Augeneinsatz nach einem der Ansprüche 1 bis 3, dadurch gekennzeichnet, daß er auch bis zu 30% Mannit umfaßt.

5. Biolöslicher, die Sicht nicht verschleiernder Augeneinsatz nach einem der Ansprüche 1 bis 4, dadurch gekennzeichnet, daß er auch ein Baschichtungspolymer, insbesondere Hydroxypropylmethylcellulosephthalat, als inneren Bestandteil umfaßt.

6. Biolöslicher, die Sicht nicht verschleiernder Augeneinsatz nach einem der Ansprüche 1 bis 5, dadurch gekennzeichnet, daß er auch eine wirksame Menge eines pharmakologisch aktiven Mittels umfaßt.

7. Biolöslicher, die Sicht nicht verschleiernder Augeneinsatz nach Anspruch 1, dadurch gekennzeichnet, daß er ungefähr 3 mg wiegt und aus 1,50 mg Polyvinylalkohol, 0,6 mg Hydroxypropylcellulose EF, 0,75 mg Mannitol und 0,15 mg Glycerin zusammengesetzt ist.

8. Biolöslicher, die Sicht nicht verschleiernder Augeneinsatz nach Anspruch 1, dadurch gekennzeichnet, daß er ungefähr 3 mg wiegt und aus 0,45 mg Polyvinylalkohol, 2,25 mg Hydroxypropylmethylcellulosephthalat und 0,30 mg Glycerin zusammengesetzt ist.

9. Biolöslicher, die Sicht nicht verschleiernder Augeneinsatz nach Anspruch 1, dadurch gekennzeichnet, daß er ungefähr 3 mg wiegt und aus 1,5 mg Polyvinylalkohol, 0,60 mg Polyvinylpyrrolidon, 0,75 mg Mannit und 0,15 mg Glycerin zusammengesetzt ist.

10. Biolöslicher, die Sicht nicht verschleiernder Augeneinsatz nach Anspruch 1, dadurch gekennzeichnet, daß er ungefähr 3 mg wiegt und aus 2,10 mg Polyvinylalkohol, 0,75 mg Mannit und 0,15 mg Glycerin zusammengesetzt ist.

11. Biolöslicher, die Sicht nicht verschleiernder Augeineinsatz nach Anspruch 1, dadurch gekennzeichnet, daß er ungefähr 3 mg wiegt und aus 1,50 mg Polyvinylalkohol, 1,35 mg Hydroxypropylmethylcellulosephthalat und 0,15 mg Glycerin zusammengesetzt ist.

## Revendications

1. Un insert oculaire biosoluble ne troublant pas la vision, caractérisé en ce qu'il comprend: au moins 15% en poids d'alcool polyvinylique ayant un poids moléculaire de 2 000 à 3 000 et de 1 à 10% en poids de glycérine.

2. Un insert oculaire biosoluble ne troublant pas la vision selon la revendication 1, caractérisé en ce qu'il comprend également jusqu'à 50% en poids d'hydroxypropylcellulose ayant un poids moléculaire de 30 000 à 100 000.

3. Un insert oculaire biosoluble ne troublant pas la vision selon l'une quelconque des revendications 1 et 2, caractérisé en ce qu'il comprend également jusqu'à 20% en poids de polyvinylpyrrolidone.

4. Un insert oculaire biosoluble ne troublant pas la vision selon l'une quelconque des revendications 1 à 3, caractérisé.en ce qu'il comprend également jusqu'à 30% de mannitol.

5. Un insert oculaire biosoluble ne troublant pas la vision selon l'une quelconque des revendications 1 à 4, caractérisé en ce qu'il comprend également un polymère de revêtement entérique, plus particulièrement le phthalate d'hydroxypropylméthylcellulose.

6. Un insert oculaire biosoluble ne troublant pas la vision selon l'une quelconque des revendications 1 à 5, caractérisé en ce qu'il comprend également une quantité efficace d'un agent pharmacologiquement actif.

7. Un insert oculaire biosoluble ne troublant pas la vision selon la revendication 1, caractérisé en ce qu'il pèse environ 3 mg et est composé de 1,50 mg d'alcool polyvinylique, 0,6 mg d'hydroxypropylcellulose EF, 0,75 mg de mannitol et 0,15 mg de glycérine.

8. Un insert oculaire biosoluble ne troublant pas la vision selon la revendication 1, caractérisé en ce qu'il pèse environ 3 mg et est composé de 0,45 mg d'alcool polyvinylique, 2,25 mg de phtalate d'hydroxypropylméthylcellulose et 0,30 mg de glycérine.

9. Un insert oculaire biosoluble ne troublant pas la vision selon la revendication 1, caractérisé en ce qu'il pèse environ 3 mg et est composé de 1,5 mg d'alcool polyvinylique, 0,60 n.g de polyvinylpyrrolidone, 0,75 mg de mannitol et 0,15 mg de glycérine.

10. Un insert oculaire biosoluble ne troublant pas la vision selon la revendication 1, caractérisé en ce qu'il pèse environ 3 mg et est composé de 2,10 mg d'alcool polyvinylique, 0,75 mg de mannitol et 0,15 mg de glycérine.

11. Un insert oculaire biosoluble ne troublant pas la vision selon la revendication 1, caractérisé en ce qu'il pèse environ 3 mg et est composé de 1,50 mg d'alcool polyvinylique, 1,35 mg de phtalate d'hydroxypropylméthylcellulose et 0,15 mg de glycérine.